Europäisches Patentamt

European Patent Office

Office européen des brevets

(19)

(11) Veröffentlichungsnummer: **0 003 524**
A1

(12)
# EUROPÄISCHE PATENTANMELDUNG

(21) Anmeldenummer: **79100197.7**

(22) Anmeldetag: **23.01.79**

(51) Int. Cl.²: **C 12 D 13/02, C 07 C 31/18**

(30) Priorität: **24.01.78 GB 281378**

(43) Veröffentlichungstag der Anmeldung: **22.08.79**
**Patentblatt 79/17**

(84) Benannte Vertragsstaaten: **BE CH DE FR GB IT NL**

(71) Anmelder: **F.Hoffmann-La Roche & Co.**
**Aktiengesellschaft, Abt. VIII-Pt, CH-4002 Basel (CH)**

(72) Erfinder: **Fujiwara, Akiko, 1059-7 Fueta, Kamakura-chi**
**Kanagawa-ken (JP)**
Erfinder: **Masuda, Setsuko, Kuritaya 5-banchi**
**Kanagawa-ku, Yokohama-shi Kanagawa-ken (JP)**
Erfinder: **Sekine, Yuzuru, Kuden-cho 250-banchi**
**Totsuka-ku, Yokohama-shi Kanagawa-ken (JP)**

(74) Vertreter: **Wuesthoff, Franz, Dr.-Ing. et al,**
**Patentanwälte Wuesthoff -v. Pechmann-Behrens-**
**Goetz Schweigerstrasse 2, D-8000 München 90 (DE)**

(54) **Verfahren zur Herstellung von D-Arabit.**

(57) Fermentative Herstellung von D-Arabit mittels Mikroorganismen der Gattung Torulopsis candida aus einer assimilierbaren Kohlenstoffquelle unter aeroben Bedingungen.

EP 0 003 524 A1

ACTORUM AG

RAN 4102/15

F. Hoffmann-La Roche & Co. Aktiengesellschaft,   Basel/Schwei

Verfahren zur Herstellung von D-Arabit

Die vorliegende Erfindung betrifft ein Verfahren zur Herstellung von D-Arabit, und zwar insbesondere ein solches Verfahren, bei welchem aus assimilierbaren Kohlenstoffquellen fermentativ unter Verwendung bestimmter Hefen selektiv D-Arabit hergestellt wird ohne gleichzeitiger Bildung anderer mehrwertiger Alkohole.

Es sind fermentative Verfahren zur Herstellung von D-Arabit bekannt, bei welchen Hefen der Gattungen Saccharomyces, Pichia, Debaryomyces und Candida verwendet wurden. Diese bekannten Verfahren sind jedoch mit verschiedenen Nachteilen behaftet. So sind bei Verwendung billiger Kohlenstoffquellen, beispielsweise Zucker oder Melasse, die Ausbeuten an D-Arabit sehr niedrig oder praktisch gleich Null. Ferner werden bei diesen Verfahren gleichzeitig andere mehrwertige Alkohole, beispielsweise Glycerin, Erythrit und dergleichen, in verhältnismässig grossen Mengen gebildet. Weiters wurde ein Verfahren zur fermentativen Herstellung von D-Arabit vorgeschlagen, bei welchem Torulopsis famata oder Candida polymorpha verwendet wird. Bei diesem Verfahren entsteht jedoch neben dem D-Arabit eine verhältnismassig grosse Menge an Erythrit. Da die Eigenschaften von Erythrit denen von D-Arabit ähnlich sind, ist bei diesem Verfahren eine aufwendige Isolierung und nachfolgende Reinigung des gewünschten D-Arabit erforderlich.

Es wurde nun überraschend gefunden, dass bestimmte Hefen, und zwar Torulopsis candida und die davon abgeleiteten Mutanten aus assimilierbaren Kohlenstoffquellen selektiv D-Arabit in sehr hohen Ausbeuten produzieren, ohne dass dabei gleichzeitig andere mehrwertige Alkohole gebildet werden.

Die vorliegende Erfindung betrifft somit ein Verfahren zur Herstellung von D-Arabit durch Kultivierung eines Mikroorganismus der Gattung Torulopsis candida, welches Verfahren es ermöglicht, aus assimilierbaren Kohlenstoffquellen unter aeroben Bedingungen in einem wässrigen Medium selektiv D-Arabit zu produzieren und diesen in einfacher Weise aus der Fermentationsbrühe zu gewinnen.

Die gemäss der vorliegenden Erfindung verwendbaren Mikroorganismen umfassen alle Stämme der Gattung Torulopsis candida, welche in der Lage sind, aus assimilierbaren Kohlenstoffquellen selektiv D-Arabit zu produzieren, sowie die davon abgeleiteten Mutanten. Diese Mutanten können aus den Mutterstämmen nach üblichen Methoden erhalten werden, beispielsweise durch Bestrahlung mit Ultraviolettlicht, Röntgenstrahlen und Gammastrahlen oder durch Behandlung mit geeigneten Mutagenen. Unter diesen Torulopsis candida Stämmen sind die folgenden bevorzugt: Torulopsis candida XY-31, isoliert aus einem Boden in Yamanashi-ken, Japan und deponiert in der Agency of Industrial Science and Technology, Fermentation Research Institute, Japan unter der Bezeichnung "FERM-P No. 4204"; Torulopsis candida XY-55 (FERM-P No. 4205) und Torulopsis candida XY-65 (FERM-P No. 4206), isoliert aus einem Boden in Saitama-ken, Japan; Torulopsis candida XY-68 (FERM-P No. 4207); Torulopsis candida XY-131 (FERM-P No. 4208), isoliert von Aepfeln; und die davon abgeleiteten Mutanten, beispielsweise Torulopsis candida XY-68-21 (FERM-P No. 4362), eine von Torulopsis candida XY-68 (FERM-P No. 4207) abgeleitete Mutante.

Die im folgenden angegebenen mykologischen Charakteristika der genannten Stämme sind fast identisch; die Stämme unterscheiden sich lediglich in der Assimilation der Kohlenstoffquelle, wie dies im folgenden angegeben wird:

(a) Wachstum

(1) Wachstum in Malz-Hefeextrakt (MY): nach 3 Tagen bei 25°C sind die Zellen kugelig bis kurz-oval, 2.0 – 7.5 μ im Durchmesser, Einzellen, in Paaren oder in kurzen Ketten. Es kann sich ein dünnes Häutchen bilden. Die Fortpflanzung erfolgt durch Sprossung.

(2) Wachstum auf MY-Agar: Nach einem Monat bei 25°C ist die Kultur grau-weiss bis cremefarbig, matt, butterartig, flach bis convex und die Kolonie hat einen kreisförmigen bis lappigen Rand.

(3) Glasplattenkultur auf Kartoffelextraktagar: Es bildet sich weder ein Mycelium noch ein Pseudomycelium.

(b) Ascosporenbildung:

Es bildeten sich Ascosporen weder auf Gorodkowaagar, noch auf Malzagar , noch auf Gipsblock.

(c) Ballistosporenbildung:

Es wurde keine Ballistosporenbildung auf MY-Agar beobachtet.

(d) Physiologische Eigenschaften:

(1) Für das Wachstum optimale Temperatur und optimaler pH:

25 - 30$^o$C, pH 4.0 - 6.5

(2) Für das Wachstum geeignete Temperatur- und pH-Bereiche:

XY-31 (FERM-P No. 4204), XY-55 (FERM-P No. 4205) und XY-65 (FERM-P No. 4206): 5 - 40$^o$C, pH 3.0 - 8.5

XY-68 (FERM-P No. 4207), XY-68-21 (FERM-P No. 4362) und XY-131 (FERM-P No. 4208): 5 - 33$^o$C, pH 3.0 - 8.5

(3) Nitratassimilierung: Negativ

(4) Fettabbau:         Negativ

(5) Harnstoffabbau:    Negativ

(6) Gelatine-Hydrolyse: Negativ

(7) Osmotoleranz:
Wachstum in einem Medium, welches 16% (Gew./Vol.) NaCl enthält.

(8) Bildung von Carotenoidpigmenten:  Negativ

5

(9) Bildung von stärkeähnlichen Verbindungen: Negativ

(10) Wachstum in vitaminfreiem Medium: schwach

(e) Assimilierung von Kohlenstoffverbindungen: Siehe
Tabelle 1

(f) Fermentation:

Bei XY-68 und XY-68-21 wurde eine schwache Glucosegärung beobachtet. Fermentation von D-Galactose, Maltose,
Rohrzucker, Lactose, Melibiose, D-Xylose, D-Arabinose,
Trehalose, Raffinose, α-Methyl-glucosid und Inulin
wurde nicht beobachtet.

**Die oben genannten Stämme wurden, außer beim Fermentation
Research Institute, auch noch bei ATCC hinterlegt, und zwar unter
den folgenden Nummern:**

| | |
|---|---|
| **FERM-P No. 4204** | **ATCC No. 20530** |
| **FERM-P No. 4205** | **ATCC No. 20531** |
| **FERM-P No. 4206** | **ATCC No. 20532** |
| **FERM-P No. 4207** | **ATCC No. 20533** |
| **FERM-P No. 4208** | **ATCC No. 20534** |
| **FERM-P No. 4362** | **ATCC No. 20535** |

Diese Hinterlegung erfolgte am 21.12.1978.

Ergänzt gemäss Antrag vom 21.2.79, eingegangen am 21.3.79.

Tabelle 1   Assimilierung von Kohlenstoffverbindungen

| | XY-31 FERM-P No. 4204 | XY-55 FERM-P No. 4205 | XY-65 FERM-P No. 4206 | XY-68 und XY-68-21 FERM-P No. 4207 und 4362 | XY-131 FERM-P No. 4208 |
|---|---|---|---|---|---|
| D-Arabinose | − | − | ± | + | + |
| L-Arabinose | + | + | + | + | + |
| D-Ribose | − | + | + | + | + |
| D-Xylose | + | + | + | + | + |
| D-Glucose | + | + | + | + | + |
| D-Galactose | + | + | + | + | + |
| L-Rhamnose | + | − | + | + | + |
| L-Sorbose | + | + | + | + | + |
| Maltose | + | + | + | + | + |
| Sucrose | + | + | + | + | + |
| Lactose | − | − | − | − | − |
| Melibiose | + | + | + | + | + |
| Cellobiose | + | + | + | + | + |
| Trehalose | + | + | + | + | + |
| Raffinose | + | + | + | + | + |
| Melezitose | + | + | + | + | + |
| $\alpha$-Methyl-D-glucosid | + | + | + | + | + |
| Lösliche Stärke | + | + | + | + | + |
| Inulin | + | + | + | + | + |
| Äthanol | + | + | + | − | + |
| Adonit | + | + | + | + | + |
| Erythrit | + | + | + | + | + |
| Inosit | − | − | − | − | − |
| D-Mannit | + | + | + | + | + |
| D-Sorbit | + | + | + | + | + |
| Glycerin | + | + | + | + | + |
| DL-Milchsäure | ± | + | + | ± | + |
| Citronensäure | + | + | + | + | + |
| Bernsteinsäure | ± | + | + | + | − |
| Salicin | + | + | + | + | + |

7

Im Hinblick auf die oben geschilderten mykologischen Charakteristika wurden die genannten sechs Stämme als Spezies von Torulopsis candida identifiziert, welche Gattung in J. Lodder, "The Yeasts, A Taxonomic Study" North Holland Publishing Company (1970) und in Iizuka und Gotoh, "Taxonomy & Identification of Yeasts" zweite Ausgabe, University of Tokyo Press (1973) beschrieben ist. Diese Stämme wurden als Torulopsis candida XY-31, XY-55, XY-65, XY-68, XY-68-21 und XY-131 bezeichnet.

Gemäss der vorliegenden Erfindung kann man aus assimilierbaren Kohlenstoffquellen selektiv D-Arabit herstellen und zwar aus assimilierbaren Kohlenstoffquellen durch Kultivierung von Mikroorganismen unter aeroben Bedingungen in einem wässrigen Medium, welche Mikroorganismen der Gattung Torulopsis candida angehören und in der Lage sind selektiv D-Arabit zu produzieren.

Die wichtigsten Ausgangsmaterialien im Kulturmedium sind assimilierbare Kohlenstoffquellen, beispielsweise Sucrose, Glucose, Fructose, Melasse, bzw. Gemische davon, welche von den verwendeten Hefen assimiliert werden können. Die bevorzugte Konzentration dieser Kohlenstoffquellen im Medium beträgt zwischen etwa 20 und 50% (Gew./Vol.)

Die für das Wachstum der verwendeten Hefen notwendigen Stickstoffquellen bestehen aus organischen Substanzen, wie beispielsweise Mais-Quellwasser, Pepton, Harnstoff, Casein-Hydrolysat, Hefeextrakt und dergleichen ; und anorganischen Substanzen, wie beispielsweise Ammoniumsulfat, Ammoniumchlorid und dergleichen. Ferner können dem Kulturmedium anorganische Salze, Vitamine und dergleichen zugesetzt werden.

Die Kultivierung kann unter aeroben Bedingungen in einem wässrigen Medium durchgeführt werden, insbesondere

8

mittels eines submersen Fermentationsverfahrens. Das Kohlenstoffquellen, Stickstoffquellen, andere Nährsubstanzquellen und anorganische Salze enthaltende Medium kann entweder direkt mit der selektiv D-Arabit produzierenden Hefe inokuliert werden oder mit einer Kulturbrühe, welche durch Vorkultivierung dieser Hefe erhalten wurde. Die Fermentation kann bei Temperaturenvon etwa 25 bis etwa 32°C durchgeführt werden. Die Fermentationszeit schwankt je nach der Art des verwendeten Mediums und der Konzentration der Kohlenstoffquellen. Im allgemeinen genügen jedoch etwa 3 bis etwa 10 Tage. Der bevorzugte pH Bereich liegt zwischen etwa 4 und etwa 7. Die Fermentation wird zweckmässig abgebrochen, sobald die maximale Ausbeute am gewünschten D-Arabit erzielt wurde. Dies kann man beispielsweise mittels Gaschromatographie, Hochdruckflüssigchromatographie und dergleichen feststellen.

Die Isolierung und Reinigung des in der Fermentationsbrühe gebildeten D-Arabit kann nach an sich bekannten Methoden durchgeführt werden, beispielsweise wie folgt: aus der erhaltenen Fermentationsbrühe werden die Zellen durch Zentrifugieren oder Filtrieren abgetrennt. Das Filtrat wird zwecks Entfernung von Verunreinigungen mit Aktivkohle behandelt und hierauf mit Methanol oder Aceton, um Proteine auszufällen. Das so erhaltene Filtrat wird durch Eindampfen völlig wasserfrei gemacht und mit heissem Äthanol extrahiert. Aus dem Extrakt lässt man den Arabit bei Raumtemperatur oder in der Kälte auskristallisieren. Nach Umkristallisierung aus einem geeigneten Lösungsmittel, beispielsweise Äthanol, erhält man reinen D-Arabit in Form von weissen Kristallen.

Der auf diese Weise in hohen Ausbeuten und ohne gleichzeitiger Bildung von anderen mehrwertigen Alkoholen erhaltene D-Arabit kann als Süssmittel verwendet werden.

## Beispiel 1

Es wird ein steriles Nährmedium hergestellt, welches 20% (Gew./Vol.) Sucrose, 1.5% Hefeextrakt (Oriental Yeast Company, Tokyo, Japan) 0.2% $(NH_4)_2SO_4$, 0.05 % $MgSO_4$, 0.05% $KH_2PO_4$ und 0.5% $CaCO_3$ enthält. Der pH-Wert dieses Mediums wird mit 5N-NaOH auf 6.5 eingestellt. 50 ml dieses Kulturmediums werden mit Torulopsis candida XY-68 (FERM-P No. 4207) in einem 500 ml Kolben angeimpft, welcher hierauf mit einem Wattepfropfen verschlossen wird. Nach 6-tägiger Inkubation bei 30°C in einer Schüttelvorrichtung (220 U/Minute) war die im Medium ursprünglich vorhandene Sucrose verschwunden und es hatten sich 4.8 g D-Arabit gebildet. Die Identifizierung des D-Arabit erfolgte mittels Dünnschicht- und Papierchromatographie und Gas-Flüssigchromatographie des Trifluoroacetatderivats.

Aus der so erhaltenen Gärbrühe werden die Zellen durch Zentrifugieren abgetrennt und das Filtrat wird über Aktivkohle geleitet und dann auf 1/5 seines Volumens eingedampft. Hierauf wird dem so erhaltenen Konzentrat ein gleiches Volumen von Aceton zugesetzt, um Proteine auszufällen. Die überstehende Flüssigkeit wird unter reduziertem Druck zu einem Sirup eingedampft. Der erhaltene Sirup wird mit heissem Äthanol extrahiert und der Extrakt gekühlt und über Nacht in der Kälte stehengelassen. Nach dem Filtrieren erhält man 4.6 g eines schwachgefärbten kristallinen Feststoffes, welcher nach Umkristallisation aus Äthanol 4.55 g weisser Kristalle mit einem Schmelzpunkt von 102.3°C und einer optischen Drehung von +7.4° in einer gesättigten Boraxlösung ergab. Das IR-Spektrum und die Retentionszeit bei der Hochdruckflüssigchromatographie waren völlig identisch mit den entsprechenden Werten einer authentischen Probe von D-Arabit.

10

In ähnlicher Weise erhielt man unter Verwendung von Torulopsis candida XY-31 (FERM-P No. 4204), XY-55 (FERM-P No. 4205), XY-65 (FERM-P No. 4206) und XY-131 (FERM-P No. 4208) D-Arabit in Mengen von 2.6 g, 3.45 g, 2.7 g und 3.0 g.

Beispiel 2

Es wird ein Nährmedium hergestellt, welches 10% Hefeextrakt, 1% $(NH_4)_2SO_4$, 0.25% $KH_2PO_4$, 0.25% $MgSO_4$ und 2.5% $CaCO_3$ enthält und dessen pH auf 6.5 eingestellt ist.

Es wurden je 15 g Sucrose, Glucose und Fructose, sowie ein Gemisch von je 7.5 g Glucose und Fructose in 500 l Kolben in Wasser gelöst und auf ein Volumen von 40 ml gebracht. Jedem Kolben wurden 10 ml der vorher im Autoklaven erhitzten Nährlösung zugesetzt. Die Kolben wurden hierauf verschlossen und sterilisiert. Der Inhalt jedes Kolbens wurde hierauf mit Torulopsis candida XY-68 (FERM-P No. 4207) angeimpft und die Kolben wurden bei 30$^{o}$C in einer Schüttel-apparatur bei 220 U/Minute inkubiert. Die mittels Gas-Flüssigchromatographie festgestellte D-Arabit-Produktion ist in der folgenden Tabelle zusammengestellt.

|  | D-Arabit (g/L) | |
| --- | --- | --- |
|  | nach 5 Tagen | nach 9 Tagen |
| Sucrose | 80 | 130 |
| Glucose | 84 | 110 |
| Fructose | 60 | 108 |
| Glucose + Fructose | 60 | 100 |

11

## Beispiel 3

1 ml einer Vorkultur von Torulopsis candida XY-68 (FERM-P No. 4207), welche bei $30^{o}C$ 2 Tage lang in einem Medium gezüchtet wurde, welches 10% (Gew./Vol.) Sucrose, 0.7% Hefeextrakt, 0.2% $(NH_4)_2SO_4$, 0.05% $MgSO_4$, 0.05% $KH_2PO_4$ und 0.5% $CaCO_3$ enthielt, wurde in einem 500 ml Kolben 50 ml eines sterilen Mediums zugesetzt, welches die folgende Zusammensetzung aufweist (%, Gew./Vol.): Melasse 40, Maisquellwasser 5, $(NH_4)_2SO_4$ 0.2, $MgSO_4$ 0.05, $KH_2PO_4$ 0.05, $CaCO_3$ 0.5 und Nissan disfoam CA-115 0.05, ein Antischäummittel hergestellt von Nippon Oils and Fats Co. Ltd., dessen Hauptbestandteile Polyalkylenglykole sind. Der anfängliche Zuckergehalt des Mediums beträgt 30.8% (Gew./Vol.), berechnet als Sucrose.

Nach einer Fermentationszeit von 8 Tagen bilden sich 108 g/L D-Arabit.

## Beispiel 4

Man stellt ein steriles Nährmedium her, welches 40% (Gew./Vol.) Melasse, 2% Hefeextrakt, 0.2% $(NH_4)_2SO_4$, 0.5% $MgSO_4$, 0.05% $KH_2PO_4$ und 0.5% $CaCO_3$ enthält. Der pH Wert dieses Mediums wird auf 6.5 eingestellt. In einem 500 ml Kolben werden 50 ml dieses Mediums mit Torulopsis candida XY-68-21 (FERM-P No. 4362) angeimpft und der Kolben wird mit einem Wattepfropfen verschlossen. Es wird in einer Schüttelvorrichtung (220 U/Min.) bei $30^{o}C$ fermentiert. Der hierbei verwendete Stamm XY-68-21 ist eine Mutante von Torulopsis candida XY-68 (FERM-P No. 4207), welcher aus letzterer durch Bestrahlung einer Zellsuspension mit einer Ultraviolettlampe in einem Abstand von 3 cm während 3 Minuten erhalten wurde. Nach 8-tägiger Inkubation ist der im ursprünglichen Medium vorhandene Zucker verbraucht und es haben sich 160 g/L D-Arabit gebildet.

Patentansprüche

1) Verfahren zur Herstellung von D-Arabit, dadurch gekennzeichnet, dass man einen Mikroorganismus der Gattung Torulopsis candida, welcher in der Lage ist, aus einer assimilierbaren Kohlenstoffquelle selektiv D-Arabit zu produzieren, unter aeroben Bedingungen in einem wässrigen Medium kultiviert und den dabei gebildeten D-Arabit aus der Fermentationsbrühe gewinnt.

2) Verfahren nach Anspruch 1, dadurch gekennzeichnet, dass man als Mikroorganismus Torulopsis candida verwendet.

3) Verfahren nach Anspruch 1 oder 2, dadurch gekennzeichnet, dass man als Mikroorganismus Torulopsis candida FERM-P No. 4207 verwendet.

4) Verfahren nach Anspruch 1 oder 2, dadurch gekennzeichnet, dass man als Mikroorganismus Torulopsis candida FERM-P No. 4362, eine Mutante von Torulopsis candida FERM-P No. 4207, verwendet.

5) Verfahren nach Anspruch 1 oder 2, dadurch gekennzeichnet, dass man als Mikroorganismus Torulopsis candida FERM-P No. 4204 verwendet.

6) Verfahren nach Anspruch 1 oder 2, dadurch gekennzeichnet, dass man als Mikroorganismus Torulopsis candida FERM-P No. 4205 verwendet.

7) Verfahren nach Anspruch 1 oder 2, dadurch gekennzeichnet, dass man als Mikroorganismus Torulopsis candida FERM-P No. 4206 verwendet.

8) Verfahren nach Anspruch 1 oder 2, dadurch gekennzeichnet, dass als Mikroorganismus Torulopsis candida FERM-P No. 4208 verwendet wird.

9) Verfahren nach einem der Ansprüche 1 - 8, dadurch gekennzeichnet, dass die Fermentation in einem wässrigen Nährmedium durchgeführt wird, welches Kohlenstoff- und Stickstoffquellen und anorganische Salze enthält.

10) Verfahren nach einem der Ansprüche 1 - 9, dadurch gekennzeichnet, dass als assimilierbare Kohlenstoffquellen Sucrose, Glucose, Fructose, Melasse oder ein Gemisch dieser Substanzen verwendet wird.

11) Verfahren nach einem der Ansprüche 1 - 10, dadurch gekennzeichnet, dass ein Nährmedium verwendet wird, in welchem die Konzentration der assimilierbaren Kohlenstoffquellen 20 - 50% (Gew./Vol.) beträgt.

12) Verfahren nach einem der Ansprüche 1 - 11, dadurch gekennzeichnet, dass die Fermentation bei einer Temperatur zwischen etwa 25°C und etwa 32°C durchgeführt wird.

13) Verfahren nach einem der Ansprüche 1 - 12, dadurch gekennzeichnet, dass die Fermentation bei einem pH-Wert von 4 bis 7 durchgeführt wird.

Europäisches Patentamt

**EUROPÄISCHER RECHERCHENBERICHT**

0003524

Nummer der Anmeldung

**EP 78 10 0855**

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| X | CHEMICAL ABSTRACTS, Vol. 59, 1963, Columbus, Ohio, USA, Nr. 1054h H. ONISHI et al.: "Fermentative production of D-arabitol and erythritol", Spalte 1054 & JP - A - 3547 ('62) Juni 5. -- | 1-13 |
| X | CHEMICAL ABSTRACTS, Vol. 77, 1972, Zusammenfassung Nr. 60051s, Columbus, Ohio, USA, H. ONISHI et al.: "Fermentative production of D-arabitol from glycerol", Seite 348, rechte Spalte & JP - A - 72 20 394, 9. Juni '72 -- | 1-9,11-13 |
| X | CHEMICAL ABSTRACTS, Vol. 66, 1967, Zusammenfassung Nr. 113259x, Columbus, Ohio, USA, YU.N. KARASEVICH: "The metabolism of D-arabinose and D-lyxose by the yeast, torulopsis candida", Seite 10515, rechte Spalte & Dokl.Akad.Nauk SSSR 173(2), 445-6 (1967)(Russ.) -- | 1-9 |
| T | J. LODDER: "The yeasts, a taxonomic study", 1974, North Holland Publishing Company, Amsterdam, Seiten 1249-1251 * Seite 1249 "Synonyms"; Seiten 1250-1251 "Discussion" * ---- | 1-8 |

**KLASSIFIKATION DER ANMELDUNG (Int.Cl.²)**

C 12 D 13/02
C 07 C 31/18

**RECHERCHIERTE SACHGEBIETE (Int. Cl.²)**

C 12 D 13/02
C 07 C 31/18

**KATEGORIE DER GENANNTEN DOKUMENTE**

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

Der vorliegende Recherchenbericht wurde für alle Patentansprüche erstellt.

| Recherchenort Den Haag | Abschlußdatum der Recherche 27-04-1979 | Prüfer DESCAMPS |
|---|---|---|

EPA form 1503.1  06.78